# EUROPEAN PATENT SPECIFICATION

(11) **EP 2 384 702 B1**
(45) Date of publication and mention of the grant of the patent: **13.07.2016**
(21) Application number: 10162229.8
(22) Date of filing: 07.05.2010
(51) Int. Cl.: B01L 9/00, G01N 1/42, A01N 1/02, A61B 10/00, B01L 9/06

(54) **Device for storing cryo-grid storage boxes**
Vorrichtung zur Aufbewahrung von Kryo-Rasteraufbewahrungsbehältern
Dispositif de stockage cryogénique des boîtes de rangement à grille

(43) Date of publication of application: 09.11.2011
(73) Proprietor: Asociación Centro de Investigación Cooperativa en Biociencias - CIC bioGUNE, 48160 Derio (ES); FUNDACIÓN IKERBASQUE, 48011 Bilbao (Biskaia) (ES)
(72) Inventor: Abrescia, Nicola Gerardo Antonio, 48160, DERIO (ES); Gil Carton, David, 48160, DERIO (ES); Chalaux, Carles, 48160, DERIO (ES)
(74) Representative: ZBM Patents - Zea, Barlocci & Markvardsen

(56) References cited:
- US-A- 4 712 607
- US-A- 4 745 771
- US-A- 5 906 101
- US-A1- 2002 084 277
- US-A1- 2004 065 093
- US-A1- 2007 228 049
- US-A1- 2008 092 581
- ANONYMOUS: 'Grid Storage Boxes - TEM, Transmission Electron Microscopy Grids', [Online] 22 December 2014, XP055159848 Retrieved from the Internet: <URL:http://www.tedpella.com/grids_html/gri dbox.htm#_160_40> [retrieved on 2014-12-22]
- LINDA MELANSON: 'Cryo TEM Cryo TEM from specimen prep to the microscope' CRYO TEM WORKSHOP BAYLOR COLLEGE OF MEDICINE, [Online] 17 October 2006, pages 1 - 33, XP055159850 Retrieved from the Internet: <URL:http://www.stehm.uvic.ca/facility/inst ruments/Cryoplunge3/Gatan_CryoTEM_presentat ion.pdf> [retrieved on 2014-12-22]
- ANONYMOUS: 'TEM grid box, grid storage box, cryo-grid box', [Online] 22 December 2014, XP055159852 Retrieved from the Internet: <URL:http://www.grid-tech.com/gridbox.htm> [retrieved on 2014-12-22]

## Description

In the field of cryo-electron microscopy, frozen samples are investigated using an electron microscope. Generally copper cryo-grids are used, in which a 3 - 4 microliter sample may be deposited. Such samples may be quickly flash-frozen using liquid ethane. Eventually, such a cryo-grid is positioned in an electron microscope to investigate the sample.

It may be necessary to store a plurality of these cryo-grids for a short or longer period of time before they can be investigated. If samples cannot be safely stored and/or may degrade at room-temperature or at around 4 degrees, the samples and the corresponding cryo-grids may need to be stored under cryogenic conditions. It is known to use cryo-grid storage boxes that comprise a plurality of cryo-grids. These cryo-grid storage boxes are then stored in dewars containing liquid nitrogen, so as to make sure the (e.g. biological) samples stay frozen during storage.

US 4, 745, 771 discloses a sample holder for the cryo-preparation of biological tissue samples for ultrastructural analysis. US 4,712,607 discloses a cryocell assembly for biological material including a space for receiving the material, the space being at least partly enclosed by means suitable for conducting heat from the space to a heat sink and including heat generating means in heat conducting relationship with the space. A power supply unit senses actual temperature in said space and compares with the desired temperature. US 2007/0228049 discloses a method and apparatus for the transportation, remote and unattended mounting, and visual alignment and monitoring protein crystals for synchrotron generated x-ray diffraction analysis. The protein samples are maintained at liquid nitrogen temperatures at all times. US 2002/084277 discloses a shipping container with an outer shipping container shell and a support assembly for holding a dewar vessel within the outer shipping container shell. The dewar vessel has an inner vessel that holds a specimen chamber and plastic foam between its inner wall and the specimen chamber. The specimen chamber allows liquid cryogen to pass through it into the plastic foam, allows liquid cryogen in a vapor phase liquid state to pass from the plastic foam into it, and acts as a filter to prevent particles or fragments of the plastic foam from entering into it. The specimen chamber is an open-celled porous thermoplastic material that is cryogenically compatible such as an aerated polypropylene foam. The plastic foam is an open cell plastic foam such as a phenolic foam.

Normally, a cryo-grid storage box is introduced into an adapted vial or container such as for example a so-called Falcon tube. Such a vial or Falcon tube is then stored in a dewar for example using a thin cord. Although this may be a practical way to store a smaller number of cryo-grid storage boxes, problems can arise when a larger number of cryo-grid storage boxes needs to be stored for a prolonged period of time. Each vial (or Falcon tube) occupies a space that is much larger than a cryo-grid storage box, and a dewar may fill up quickly when using a plurality of vials. This may mean that more dewars or larger dewars need to be used. Another problem that may occur is that with a plurality of vials (or Falcon tubes), it may not be straightforward to trace each sample.

There thus exists a need for a device that is suitable for storing samples to be investigated using cryo-electron microscopy which can help to reduce the need for dewar storage space. There also exists a need for such a device which can be easily handled using conventional and commercially available laboratory tools. There furthermore exists a need for a device which may facilitate the tracking and/or identification of individual samples.

It is an object of the present invention to at least partially fulfil one or more of these needs.

In a first aspect, the invention provides a device for storing a plurality of cryo-grid storage boxes in a dewar, comprising a cylindrical body having a diameter, wherein said diameter is such that the device fits substantially exactly in a dewar canister, and said cylindrical body comprises a plurality of storage holes, the cross-sectional dimensions of the storage holes being adapted to a standard-size cryo-grid storage box such that each storage hole fits one or more standard-size cryo-grid storage boxes stacked on top of each other. Herein, the standard-size cryo-grid storage box is a circular storage box having a diameter of approximately 13 mm, or a rectangular storage box having a length of approximately 13 mm and a width of approximately 13 mm.

A plurality of ordinary, commercially available cryo-grid storage boxes may thus be introduced into the plurality of storage holes provided in a cylindrical body. The diameter of the cylindrical body may be such that it substantially exactly fits in a standard size dewar canister. This way, a plurality of samples may be stored in an efficient manner.

In some embodiments, the cylindrical body may comprise nine storage holes. However, other numbers of storage holes may be used. The ideal number of storage holes may depend e.g. on the size and shape of the cryo-grid storage boxes used in a laboratory.

In some embodiments, the depth of at least one storage hole is such that two or more cryo-grid storage boxes may be stored on top of each other. Each hole may thus comprise more than one cryo-grid storage box stacked on at least one other storage box. Each storage hole may thus e.g. be used to store cryo-grid storage boxes with similar samples, or samples from the same "owner".

In some embodiments, an identifier may be provided in proximity to each storage hole. An identifier may be used to identify an "owner" of a certain sample, or to identify an individual sample. The identifier may comprise e.g. a number or a letter, or a symbol. In these embodiments, a plurality of samples from different owners may thus be stored in a single cylindrical body, and each sample still may be identified easily. The identifiers may be either removably attached to the storage device, or they may be formed integrally with the cylindrical body.

In some embodiments, the storage holes in the cylindrical body may have a circular cross-section. The dimensions and shape of the holes may be adapted to commercially available circular cryo-grid storage boxes. In other embodiments, the storage holes may have a square cross-section and be adapted to the size of commercially available square cryo-grid storage boxes.

In some embodiments, at least one storage hole may have different dimensions or a different shape than one other hole. Such a device may thus be used to store cryo-grid storage boxes of different shapes.

In some embodiments, the cylindrical body furthermore comprises a pin by which the body may be handled. Using commercially available tweezers, clamps or similar conventional tools, the device may thus be easily introduced into a canister. Several or a unique indentation in the pin may be provided along the pin to facilitate these operations. Said canister may then be used to lower the samples into a dewar. When wanting to retrieve a sample, a canister may be used to lift the device out of the dewar. Using the pin, the device can be easily retrieved from the canister.

Optionally, said cylindrical body comprises a central recess and said pin is provided in said central recess. Optionally, said plurality of storage holes may be provided around said central recess.

Particular embodiments of the present invention will be described in the following, only by way of non-limiting examples, with reference to the appended drawings, in which:
Figures 1 a and 1 b illustrate a first embodiment of a storage device according to the present invention;
Figures 2a and 2b illustrate a second embodiment of a storage device according to the present invention;
Figure 3 illustrates a further embodiment of a storage device according to the present invention;
Figure 4 illustrates yet a further embodiment of a storage device according to the present invention;
Figure 5 shows an isometric view and a cross-sectional view of a dewar which may be used in combination with embodiments of the present invention;
Figure 6 shows top views of two commercially available cryo-grid storage boxes which may be used advantageously in combination with embodiments of the present invention.

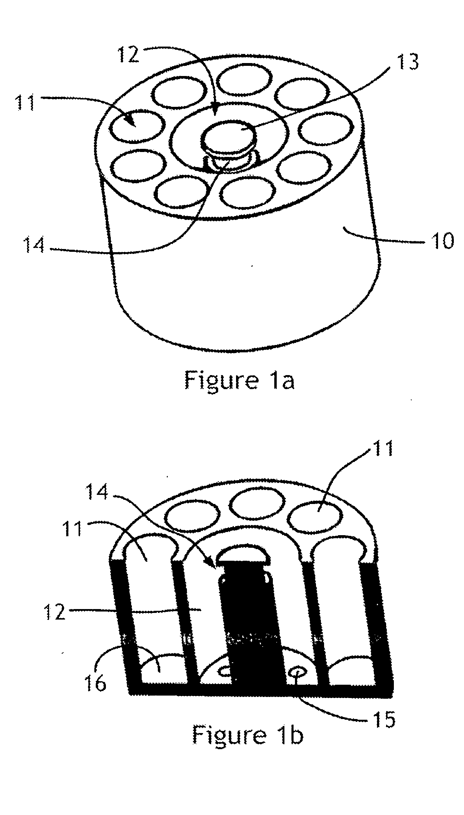

Figure 1a illustrates a first embodiment of a storage device 10. This storage device 10 comprises nine holes 11 of suitable dimensions such that each hole fits a commercially available cryo-grid storage box. Cryo-grid storage boxes are known of various dimensions and various shapes.

It is convenient for cryo-grid storage boxes if they can be handled automatically using robot machinery or other standardized electron microscopy laboratory equipment. The storage boxes therefore may generally be of standardized dimensions. For example, Tedpella ™ commercially offers circular cryo-grid storage boxes. Each storage box is able to contain four (biological) samples which may need to be studied using electron microscopy.

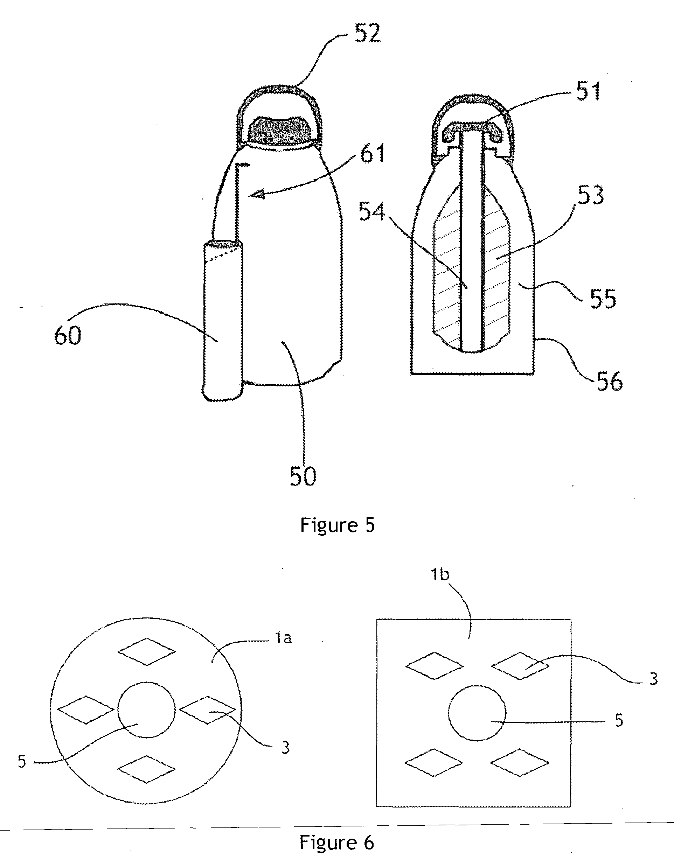

An example of a circular cryo-grid storage box is shown in figure 6. Storage box 1 a comprises four receptacles 3 in which a sample may be introduced. The storage box 1a may further comprise a protruding part 5, which may e.g. be a bolt, screw or pin. Such a bolt 5 may be adapted to be handled using laboratory equipment such as a magnetic handling rod, tweezers, clamps or other. Preferably, bolt 5 may be magnetic which makes the storage boxes particularly easy to handle using e.g. a magnetic rod. Cryo-grid storage boxes of this type are both known with and without a top lid.

Such a circular storage box has a diameter of approximately 13 mm and a height of approximately 7 mm (including the head of the screw or bolt 5); Storage holes 11 of the device are adapted to such a standard sized storage box, and may have a diameter of approximately 14 mm. It will be clear that a slightly different diameter may also be used; the diameter of the storage hole may be determined such that they are able to receive and contain a storage box, and allows easy introduction and extraction. If a much larger diameter is chosen, less cryo-grid storage boxes may be stored in the device, reducing the efficiency of the solution.

The device further comprises a recess 12, in which a pin 13 is provided. Such a pin 13 may facilitate handling of the device using conventional laboratory tools, such as e.g. tweezers, clamps, or tongs. To further facilitate this handling, an indentation 14 may be provided.

Figure 1b shows a cross-sectional view of device 10 according to figure 1 a. Bottom 16 of device 10 comprises a plurality of through-holes 15, such that upon introduction in a dewar, liquid nitrogen can pass through these holes and fill up the device.

In the embodiment of figure 1a, the depth of the hole may be e.g. approximately 40mm. This way, a plurality of cryo-grid storage boxes (for example one to four storage boxes) may be stacked within each hole 11. In this embodiment, the device 10 may store e.g. thirty-six cryo-grid storage boxes. Compared to using individual vials (or Falcon-tubes), the required space is significantly reduced, and the use of dewars in laboratories may be optimized. It will be clear that the height of the storage device 10 and depth of storage holes 11 may be varied in accordance with circumstances.

Although not shown in figure 1, an identifier may be provided in the proximity of each of the storage holes. Such an identifier may e.g. comprise numbers or letters. Such identifiers may help to be able to distinguish between various samples e.g. belonging to different providers, or belonging to different people working in the same laboratory and using the same equipment.

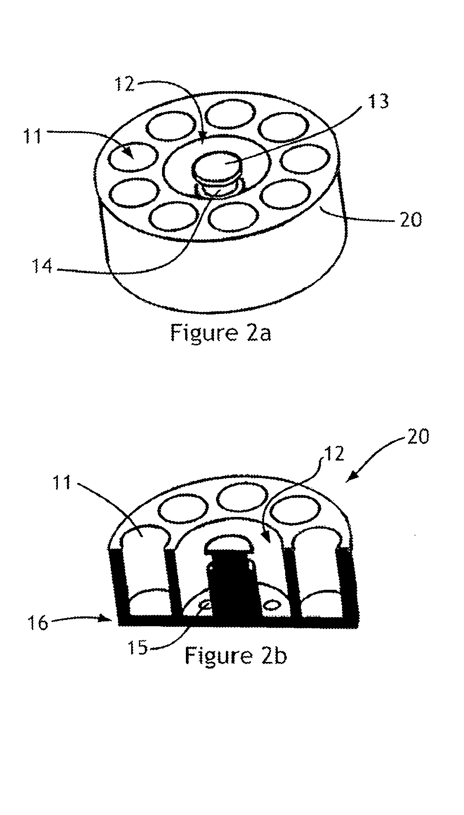

Figures 2a and 2b show an isometric view and a cross-sectional view of a second embodiment of a storage device according to the present invention. The difference between the embodiment of figure 2 and the embodiment of figure 1 is that the height of the device 20 is smaller. Also, the depth of holes 11 is reduced as compared to the previous embodiment. Instead of three or four circular storage-boxes, only two storage boxes can be stacked in each storage hole. On the other hand, more storage devices can be stacked within a canister and stored within the same dewar. Also in this embodiment, a pin 13 having an indentation 14 is provided in a recess 12. Also, through-holes 15 are provided in a central part of the bottom 16.

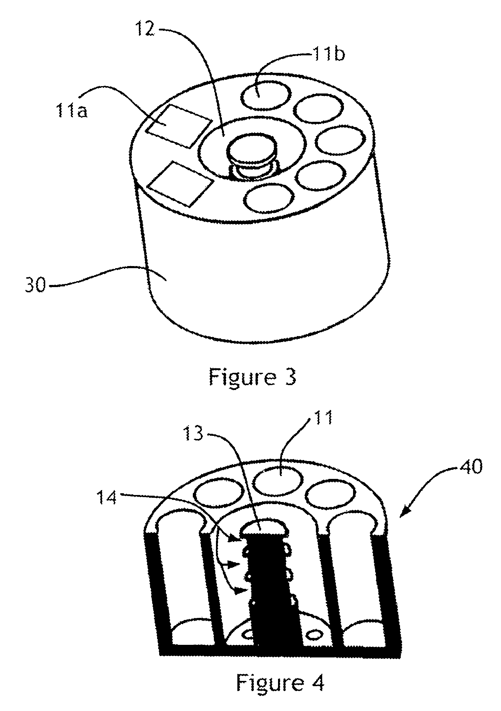

Figure 3 illustrates a further embodiment of a storage device according to the present invention. Storage device 30 comprises storage holes 11 a adapted to fit one or more rectangular storage boxes and storage holes 11 b adapted to fit one or more circular storage boxes. Once again, a central recess 12 is provided with a pin. In this embodiment, two rectangular storage holes 11a and five circular storage holes are provided. In other embodiments, the ratio of rectangular and circular storage holes could be different. In yet further embodiments, only rectangular holes could be provided.

Figure 6 shows a top view of an example of a rectangular cryo-grid storage box commercially available from Tedpella ™. A cryo-grid storage box 1 b may comprise a plurality of apertures 3 in which samples may be introduced. Cryo-grid storage boxes of this type are also both known with and without a top lid. The length and width of such a rectangular storage box are approximately 13 x 13 mm. The corresponding dimensions of storage hole 11 b may be approximately 14 x 14 mm. It will be clear that slightly different dimensions may also be used, as long as a storage box can be easily introduced, stored and extracted. Choosing too large dimensions will reduce the efficiency of the storage device.

Figure 4 illustrates a storage device 40 of similar configuration as figure 1. However, three indentations 14 are provided along the pin 13. The plurality of indentations may further facilitate the handling of the device using ordinary laboratory equipment, e.g. tweezers. In the embodiments shown so far, indentations were provided along a pin provided in a central recess. However, in other embodiments, a central pin may be provided with one or more annular protrusions instead of indentations. In alternative embodiments, the central recess may be provided e.g. with annular rims to facilitate handling. In yet further embodiments, no central pin or recess is provided and instead the device is foreseen of e.g. a magnetic portion which may facilitate handling. In yet other embodiments, the device may comprise a central threaded hole for handling with a tool having a mating thread.

Figure 5 shows an isometric view and a cross-sectional view of a commercially available dewar 50 for storing samples in liquid nitrogen. Also shown in figure 5 is a canister 60 which may be used to insert samples into and extract samples from dewar 50. Dewars 50 may generally comprise a handle 51, a lid 52 and an exterior high-strength metal body with a central tubular opening 54. Around said tubular opening 54, adsorption material 53 may be provided. Between the adsorption material and housing 56, a vacuum 55 may be established. Using such a construction, samples can be effectively stored in liquid nitrogen and heat transfer either through transmission, convection or conduction is avoided. Such dewars are commercially available from e.g. Taylor-Wharton Cryogenics ™.

In use of the device, one or more storage devices according to the present invention may be introduced into a canister 60. Such a storage device is adapted to fit substantially exactly in said canister. The diameter of the storage device may be e.g. approximately 65 mm. For ease of introduction, a top part of a canister may be adjusted by a diagonal cut (as schematically indicated with an interrupted line in figure 5). In other embodiments of the invention, the diameter of the cylindrical body of the storage device may be reduced slightly so as to facilitate its introduction in a canister.

Canister 60 fits substantially exactly in tubular opening 54 of dewar 50 filled with liquid nitrogen. When a sample stored in a cryo-grid storage box is to be examined, canister 60 may be removed from dewar 50; one or more storage devices can be extracted from the canister using e.g. tweezers. Subsequently, a sample may be taken from the storage device.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described before, but should be determined only by a fair reading of the claims that follow.

## Claims

1. A device (10; 20) for storing a plurality of cryo-grid storage boxes (1a, 1b) in a dewar (50), comprising a cylindrical body having a diameter, wherein
said diameter is such that the device fits substantially exactly in a dewar canister (60), and
said cylindrical body comprises a plurality of storage holes (11; 11a, 11 b), the cross-sectional dimensions of the storage holes (11; 11 a, 11 b) being adapted to a standard-size cryo-grid storage box (1a, 1b) such that each storage hole (11; 11 a, 11 b) fits one or more standard-size cryo-grid storage boxes (1 a, 1 b) stacked on top of each other, **characterised in that**
the standard-size cryo-grid storage boxes are circular storage boxes (1a) having a diameter of approximately 13 mm, or rectangular storage boxes (1 b) having a length of approximately 13 mm and a width of approximately 13 mm.

2. A device according to claim 1, wherein said cylindrical body comprises nine storage holes (11).

3. A device according to claim 1 or 2, wherein the depth of at least one storage hole is such that two or more cryo-grid storage boxes (1 a, 1 b) may be stored on top of each other.

4. A device according to any of claims 1 - 3, wherein an identifier is provided in proximity of each storage hole (11; 11 a, 11 b).

5. A device according to any previous claim, wherein one or more of said storage holes (11; 11b) have a circular cross-section.

6. A device according to any previous claim, wherein one or more of said storage holes (11 a) have a square cross-section.

7. A device according to any previous claim, wherein at least one storage hole has different dimensions or a different shape than one other storage hole.

8. A device according to any previous claim, wherein said body furthermore comprises a pin (13) by which the body may be handled.

9. A device according to claim 8, wherein said cylindrical body comprising a central recess (12) and said pin (13) is provided in said central recess (12).

10. A device according to claim 9, wherein said pin (13) comprises one or more indentations (14) to facilitate its handling.

11. A device according to claim 9 or 10, wherein said plurality of holes is provided around said central recess (12).

12. A device according to any of claims 9 - 11, wherein the bottom (16) along a portion of said central recess comprises one or more through-holes (15).

13. A device according to any of claims 1 - 8, wherein the cylindrical body comprises one or more through-holes (15).

## Patentansprüche

1. Eine Vorrichtung (10; 20) zur Aufbewahrung von einer Vielzahl von Kryo-Rasteraufbewahrungsbehältern (1 a, 1 b) in einem Dewargefäß (50), umfassend einen zylindrischen Körper, der einen Durchmesser hat, wobei
der Durchmesser derart ist, dass die Vorrichtung im Wesentlichen genau in einem Dewarkanister (60) passt, und
der zylindrische Körper eine Vielzahl von Aufbewahrungsbohrungen (11; 11 a, 11 b) umfasst, wobei die Querschnittabmessungen der Aufbewahrungsbohrungen (11, 11 a, 11b) an einen Kryo-Rasteraufbewahrungsbehählter (1 a, 1 b) von Standardgröße angepasst ist, so dass jede Aufbewahrungsbohrung (11; 11 a, 11 b) an einen oder mehrere aufeinander gestapelte Kryo-Rasteraufbewahrungsbehälter (1 a, 1 b) von Standardgröße angepasst ist, **dadurch gekennzeichnet, dass**
die Kryo-Rasteraufbewahrungsbehälter von Standardgröße kreisförmige Aufbewahrungsbehälter (1 a) sind, die einen Durchmesser von etwa 13 mm haben, oder rechteckige Aufbewahrungsbehälter (1 b) sind, die eine Länge von etwa 13 mm und eine Breite von etwa 13 mm haben.

2. Eine Vorrichtung nach Anspruch 1, wobei der zylindrische Körper neun Aufbewahrungsbohrungen (11) umfasst.

3. Eine Vorrichtung nach Anspruch 1 oder 2, wobei die Tiefe von mindestens einer Aufbewahrungsbohrung derart ist, dass zwei oder mehr Kryo-Rasteraufbewahrungsbehälter (1 a, 1 b) aufeinander aufbewahrt werden können.

4. Eine Vorrichtung nach einem der Ansprüche 1 - 3, wobei ein Identifikator in der Nähe von jeder Aufbewahrungsbohrung (11; 11 a, 11 b) bereitgestellt ist.

5. Eine Vorrichtung nach einem der vorhergehenden Ansprüche, wobei eine oder mehr von den Aufbewahrungsbohrungen (11; 11 b) einen kreisförmigen Querschnitt haben.

6. Eine Vorrichtung nach einem der vorhergehenden Ansprüche, wobei eine oder mehr von den Aufbewahrungsbohrungen (11a) einen rechtwinkeligen Querschnitt haben.

7. Eine Vorrichtung nach einem der vorhergehenden Ansprüche, wobei mindestens eine Aufbewahrungsbohrung verschiedene Abmessungen oder eine verschiedene Form als eine andere Aufbewahrungsbohrung hat.

8. Eine Vorrichtung nach einem der vorhergehenden Ansprüche, wobei der Körper weiterhin einen Stift (13) umfasst, durch den der Körper gehandhabt werden kann.

9. Eine Vorrichtung nach Anspruch 8, wobei der zylindrische Körper eine zentrale Vertiefung (12) umfasst und den Stift (13) in der zentralen Vertiefung (12) bereitgestellt ist.

10. Eine Vorrichtung nach Anspruch 9, wobei der Stift (13) eine oder mehrere Einbuchtungen (14) umfasst, um dessen Handhabung zu ermöglichen.

11. Eine Vorrichtung nach Anspruch 9 oder 10, wobei die Vielzahl von Bohrungen um die zentrale Vertiefung (12) herum angeordnet sind.

12. Eine Vorrichtung nach einem der Ansprüche 9 - 11, wobei der Boden (16) entlang eines Teiles der zentralen Vertiefung eine oder mehrere Durchgangsbohrungen (15) umfasst.

13. Eine Vorrichtung nach einem der Ansprüche 1 - 8, wobei der zylindrische Körper eine oder mehrere Durchgangsbohrungen (15) umfasst.

## Revendications

1. Un dispositif (10 ; 20) de stockage d'une pluralité de boîtes de stockage cryogénique de rangement à grille (1a, 1b) dans un dewar (50), comprenant un corps cylindrique ayant un diamètre, dans lequel
ledit diamètre est tel que le dispositif s'adapte essentiellement de façon exacte à un réservoir dewar (60), et
ledit corps cylindrique comprend une pluralité de trous de stockage (11 ; 11a, 11 b), étant les dimensions transversales des trous de stockage (11 ; 11a, 11 b) adaptées à une boîte de stockage cryogénique de dimensions standard (1a, 1b) de façon que chaque trou de stockage (11 ; 11a, 11 b) s'adapte à une ou à plusieurs boîtes de stockage cryogénique de dimensions standard (1a, 1b) empilées les unes sur les autres,
**caractérisé en ce que**
les boîtes de stockage cryogénique de rangement à grille de dimensions standard sont des boîtes de stockage circulaires (1 a) ayant un diamètre d'environ 13 mm, ou des boîtes de stockage rectangulaires (1 b) ayant une longueur d'environ 13 mm et une largeur d'environ 13 mm.

2. Un dispositif selon la revendication 1, dans lequel ledit corps cylindrique comprend neuf trous de stockage (11).

3. Un dispositif selon la revendication 1 ou 2, dans lequel la profondeur d'au moins un trou de stockage est tel que deux ou plusieurs boîtes de stockage cryogénique (1 a, 1 b) peuvent être stockées empilées les unes sur les autres.

4. Un dispositif selon l'une quelconque des revendications 1 - 3, dans lequel un identificateur est fourni à proximité de chaque trou de stockage (11 ; 11 a, 11 b).

5. Un dispositif selon l'une quelconque des revendications antérieures, dans lequel un ou plusieurs desdits trous de stockage (11 ; 11 b) ont une section transversale circulaire.

6. Un dispositif selon l'une quelconque des revendications antérieures, dans lequel un ou plusieurs desdits trous de stockage (11a) ont une section transversale carrée.

7. Un dispositif selon l'une quelconque des revendications antérieures, dans lequel au moins un trou de stockage a des dimensions différentes ou une forme différente qu'un autre trou de stockage.

8. Un dispositif selon l'une quelconque des revendications antérieures, dans lequel ledit corps comprend en outre une goupille (13) moyennant laquelle le corps peut être manipulé.

9. Un dispositif selon la revendication 8, dans lequel le corps cylindrique comprend une cavité centrale (12) et ladite goupille (13) est fournie dans ladite cavité centrale (12).

10. Un dispositif selon la revendication 9, dans lequel ladite goupille (13) comprend une ou plusieurs encoches (14) afin de faciliter sa manipulation.

11. Un dispositif selon la revendication 9 ou 10, dans lequel ladite pluralité de trous est fournie autour de ladite cavité centrale (12).

12. Un dispositif selon l'une quelconque des revendications 9 - 11, dans lequel le fond (16) le long d'une partie de ladite cavité centrale comprend un ou plusieurs trous passants (15).

13. Un dispositif selon l'une quelconque des revendications 1 - 8, dans lequel le corps cylindrique comprend un ou plusieurs trous passants (15).
